# EUROPÄISCHE PATENTANMELDUNG

(11) **EP 0 591 623 A2**
(43) Veröffentlichungstag der Anmeldung: **13.04.1994**
(21) Anmeldenummer: 93110613.2
(22) Anmeldetag: 02.07.1993
(51) Int. Cl.: G01N 33/24, G01N 33/00, G01N 27/00, G01T 1/16

(54) **System zur Erkennung und Überwachung von Schadstoffen**

(30) Priorität: 05.10.1992 DE 4233457; 15.02.1993 DE 4304521
(71) Anmelder: Alfred Kunz GmbH & Co., D-80336 München (DE)
(72) Erfinder: Pallor, Hans Fritz, Dipl.-Ing., D-68165 Mannheim 1 (DE)
(74) Vertreter: Laufhütte, Dieter, Dr.-Ing.

(57) **Zusammenfassung**

Die Erfindung betrifft ein System zur Erkennung und Überwachung von Schadstoffen, bei dem in mindestens einer Aufnahmekammer Kompakt-Mikrotechnik-Kombinationen bestehend aus Sensorelementen für verschiedene Überwachungsfunktionen mit sämtlichen dazugehörigen mikroelektronischen Komponen angeordent sind. Über ein Bussystem können die Daten schnell hintereinander abgefragt werden, so daß eine permanente Überwachung, beispielsweise einer Basisabdichtung von Deponien ermöglicht ist.

## Beschreibung

Die Erfindung betrifft ein System zur Erkennung und Überwachung von Schadstoffen.

Ein Beispiel für die Problematik der Erkennung und Überwachung von Schadstoffen kann anhand der Deponie-Abdichtung erläutert werden. Bei bekannten Systemen zur Altlasten-Basisabdichtung im Schwertvortriebverfahren mit nachgeordnetem Abdichtungsbahneinzug und Hilfs- bzw. Überströmkanalisation zur Abschottung von Altlasten, Deponien und neuen Deponieanlagen besteht bislang keine Überprüfbarkeit von folgenden Faktoren:
a) Dichtigkeit der Abschottung (Folie) über die Zeit;
b) Reaktionen des Deponiegutes durch chemische Abläufe und physikalische Veränderungen des Deponiegutes;
c) Erfassung, Auswertung und frühzeitige Einleitung von Gegenmaßnahmen zur Verhinderung von Verseuchung von beispeilsweise unter der Deponie befindlichem Grundwasser.

Neu angelegte Deponien und/oder zu sanierende Altlastenlager sind gegen die geologische Umgebung hermetisch abzuschotten, um das Eindringen von Schadstoffen in die Umgebung zu vermeiden. Die Abschottung erfolgt über Abschottungssperrfolien und Mehrkammerschotts.

Bei der Überwachung der Funktionsfähigkeit derartiger Deponien ergibt sich eine sehr komplexe Problemstellung. Hier muß gleichzeitig das mechanische Langzeitverhalten der Abschottung und das Verhalten der chemischen Prozesse in der Deponie vor und nach der Sanierung untersucht werden. Weiterhin müssen die Verläufe von chemisch biologischen Abbaufortschritten, d.h. der Umwandlung der Schadstoffe, analysiert werden. Schließlich ist ein Frühwarnsystem bei Abschottungsveränderungen (Erkennen von Deformation bis hin zur Beschädigung) einzurichten. Schließlich gehört auch in den Problemkreis ein Frühwarnsystem bezüglich der Einlagerung bzw. dem Freiwerden von Strahlen.

Auch in anderen Bereichen ergeben sich Probleme bei der Erkennung und Überwachung von Schadstoffen. So ist es beispielsweise wünschenswert zumindest qualitative Aussagen über die Schadstoffzusammensetzung in Müllcontainern, Rohrleitungen oder im kontaminierten Boden von alten Industrieanlagen - sogenannten Altlasten zu erhalten.

Aufgabe der vorliegenden Erfindung ist es nun, ein System zur Erkennung und Überwachung von Schadstoffen an die Hand zu geben, mit dem die Schadstoffe bezüglich der zuvor genannten wesentlichen Faktoren zuverlässig erkannt und gegebenenfalls kontinuierlich überwacht werden kann.

Erfindungsgemäß wird die Aufgabe durch die Merkmalskombination des kennzeichnenden Teils des Anspruchs 1 gelöst. Nach dieser Lösung sind in mindestens einer Aufnahmekammer ein oder mehrere Sensorelemente für die verschiedenen Überwachungsaufgaben angeordnet. Innerhalb der Aufnahmekammern ist jeweils dem Sensorelement nachgeschaltet eine kompakte Mikroelektronikbaueinheit mit Signalanpassung, Signalverarbeitung und erster Schnittstelle angeordnet. Über eine zweite Schnittstelle ist eine Datenverarbeitungsanlage zur Auswertung, Aktoraussteuerung angeschlossen und es sind entsprechende Aktorelemente anschließbar. Zwischen der ersten und zweiten Schnittstelle ist ein Bussystem angeordnet, mit dem die Systeme der einzelnen Aufnahmekammern mit der Datenverarbeitungsanlage gekoppelt sind.

Die Sensoren können in vorteilhafter Ausgestaltung der Erfindung mit Sensorschutzhauben abgedeckt sein. Hierdurch wird ein Schutz vor direkter Wassereinwirkung gegeben.

In dem System sind Sensoren zur Erfassung der Temperatur, des Drucks, der Feuchte, der Leitfähigkeit, der Radioaktivität und/oder zur Analyse von Gas vorhanden, die taktweise nacheinander abfrabgar sind.

Als geschlossene Stabsensoren können Sensoren zum Einsatz kommen, die modulierte Magnetfelder mit Aussagedifferenzierung über die Veränderung bzw. Beeinflussung der Magnetfelder zulassen.

Die erste und zweite Schnittstelle können einen Glasfaser-Optokopplersystem umfassen.

Das System kann zur Überwachung von Deponien mit einer Basis-Abdichtung, vorzugsweise einer Mehrkammerabschottungssperrfolie, eingesetzt werden. In diesem Fall sind mehrere Aufnahmekammern für die Sensoren verteilt über die Basis- Abdichtung angeordnet und in diese integriert.

Das System kann auch zur Überwachung von Deponien mit einer Oberflächenabdeckung verwendet werden, wobei die Aufnahmekammern verteilt über die Oberflächenabdeckung angeordnet und in diese integriert sind.

Andererseits kann das System auch in einen Müllcontainer bzw. Müllbehältnis zur Identifikation der Zusammensetzung des Abfalls angeordnet sein. Hierdurch ist eine Identifikation der Zusammensetzung des Abfallgutes vor der Entsorgung auf der Deponie möglich.

Mehrere Sensorelemente können gemäß einer alternativen Ausführungsform in einem vorzugsweise zylindrischen Sensorgehäuse zusammen mit der entsprechenden Elektronik angeordnet sein. Hier kann eine Sensorbestückung zur Erfassung von Feuchte, lonenkonzentration, Druck, Strahlung und Temperatur usw. integriert sein. Das gekapselte Sensorgehäuse kann in ein entsprechend vorgetriebenes Bohrloch absenkbar sein, um mit dem System in dem entsprechenden Bohrloch Daten über u.a. die Feuchte, lonenkonzentration, Druck, Strahlung und Temperatur vor Ort zu erfassen. Damit lassen sich qualitative Analyse Ergebnisse beispielsweise in ungeschützen Altlasten unmittelbar vor Ort ermitteln. Hier ist es also im Unterschied zu der bislang notwendigen Verfahrensweise nicht mehr notwendig, eigene Proben zu übernehmen, die dann in einem Labor analysiert werden müssen. Es können mit diesem vorteilhaft ausgestalteten System sofort Aussagen zuverlässig gemacht werden. Dabei ist es besonders vorteilhaft, eine tragbare elektronische Auswerteeinheit, beispielsweise ein Notebook, anzuschließen.

Gemäß einer weiteren Ausführungsform kann das System in einer Spund- oder Schlitzwand innerhalb eines Tunnelkörpers angeordnet sein, um Druckwasseransammlungen und Veränderungen des Gesteins- und Verfüllmaterials zu erkennen. Hier kann das System zum Controlling und als Frühwarnsystem zur Schadensverhinderung und Langzeitüberwachung Einsatz finden.

Das System läßt sich auch in einem Kanalsanierungsrobotter integrieren.

Schließlich läßt sich das System auch unterhalb von wasserführenden Kanälen und Rohren anordnen, um austretendes Abwasser frühzeitig zu erkennen und zu analysieren.

Die Stromversorgung der Elektronik und Sensorik läßt sich über spezielle Multikabelanbindungen sicherstellen. Für Datenübermittlungen können in üblicher Weise Meßdatenkabel eingesezt werden. Alternativ kann die Stromversorgung auch über Langzeitakkummulatoren erfolgen. Die Sensorikmeßdaten können anstatt über ein Meßdatenkabel auch über Funk im Intervall abgerufen werden. Dabei stellen die Sensorelemente die passiven Bauteile dar, während Sender und Empfänger den aktiven Teil darstellen.

Weitere Einzelheiten und Vorteile des erfindungsgemäßen Systems werden anhand eines speziellen Ausführungsbeispiels, das sich nur auf eine Anwendungsvariante des unterschiedlich einsetzbaren Systems bezieht, anhand der Zeichung und mehrerer Diagramme im folgenden näher erläutert. Es zeigen:
Fig. 1: eine schematische Übersicht eines Teils des erfindungsgemäßen Systems,
Fig. 2: ein Prinzipblockschaltbild der Übersichtsdarstellung gemäß Fig. 1,
Fig. 3: ein ausführliches Blockschaltbild des Systemteils gemäß Fig. 1,
Fig. 4: ein Prinzipblockschaltbild des gesamten erfindungsgemäßen Systems,
Fig. 5: ein Diagramm bezüglich des prinzipiellen Aufbaus des Bussystems und
Fig. 6: eine Übersicht des Systemaufbaus.

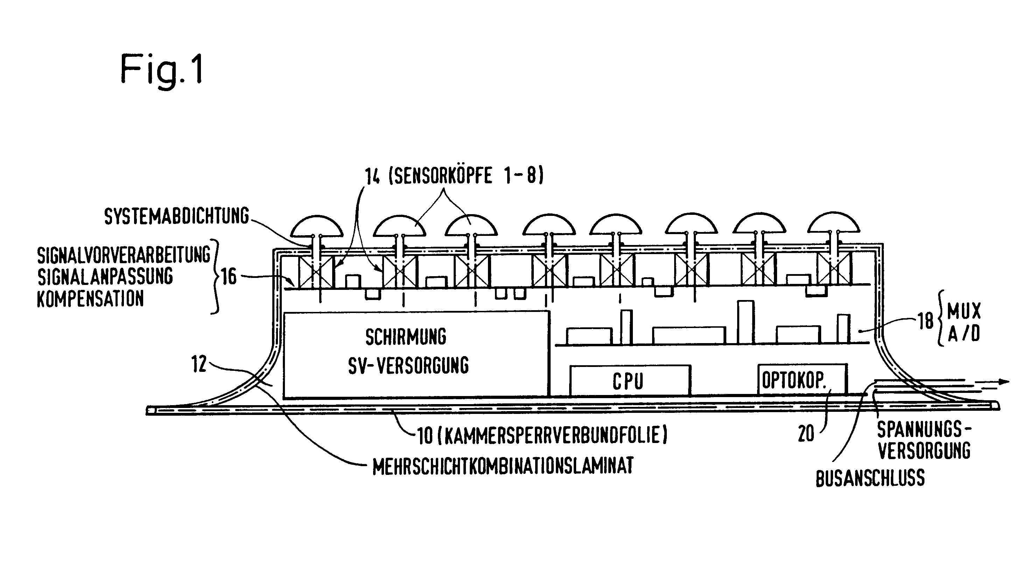

In Fig. 1 ist als Basis-Abdichtung eine Kammersperrverbundfolie 10 vorgesehen. In dieser ist eine Aufnahmekammer 12 angeordnet, deren Außenwandung aus einem Mehrschichtkombinationslaminat geformt ist. Innerhalb der Aufnahmekammer sind Sensorelemente 14 angeordnet, denen eine kompakte Mikroelektronikbaueinheit nachgeschaltet ist. Diese besteht zunächst einmal aus einer Schaltung für die Signalvorverarbeitung, Signalanpassung und Kompensation 16, einer nachgeschalteten Einheit aus Multiplexern und A/D-Wandlern 18 und einer Zentraleinheit CPU sowie einer ersten Schnittstelle 20 mit einem Optokoppler. Innerhalb der Aufnahmekammer 12 ist auch noch eine Schirmung und eine Spannungsversorgung angeordnet. Die Sensorköpfe der Sensorelemente 14 ragen, wie hier dargestellt, über die Aufnahmekammer 12 heraus, wobei an der Austrittstelle eine entsprechende Abdichtung angebracht ist.

Die Aufnahmekammer kann in nicht näher dargestellter Art und Weise ein Schutzgehäuse aus Polyethylen zur inneren Aufnahme der Mikroelektronik enthalten. Die verschiedenen Sensorköpfe können mit einer entsprechenden wasserdichten Durchdringung durch die Aufnahmekammer geführt sein. Die Sensoren können alternativ auch außen am Gehäuse integriert sein. Es sind wasser- und bruchfeste Ein- und Ausgänge zur Energieeinspeisung und Zuführungen der Sensorausgänge zur Mikroplatine vorhanden. Das Schutzgehäuse kann aus Polyethylen-Vollmaterial gefräst sein und dichte Seiten/Stirneingänge in Wendelsteckteilen aufweisen. Zusätzliche abgeflachte Seiten- und Stirnteile dienen zur statischen Lastverteilung. Zusätzlich können Befestigungsaufnahmeflächen zur Bahnfolie 10 vorhanden sein.

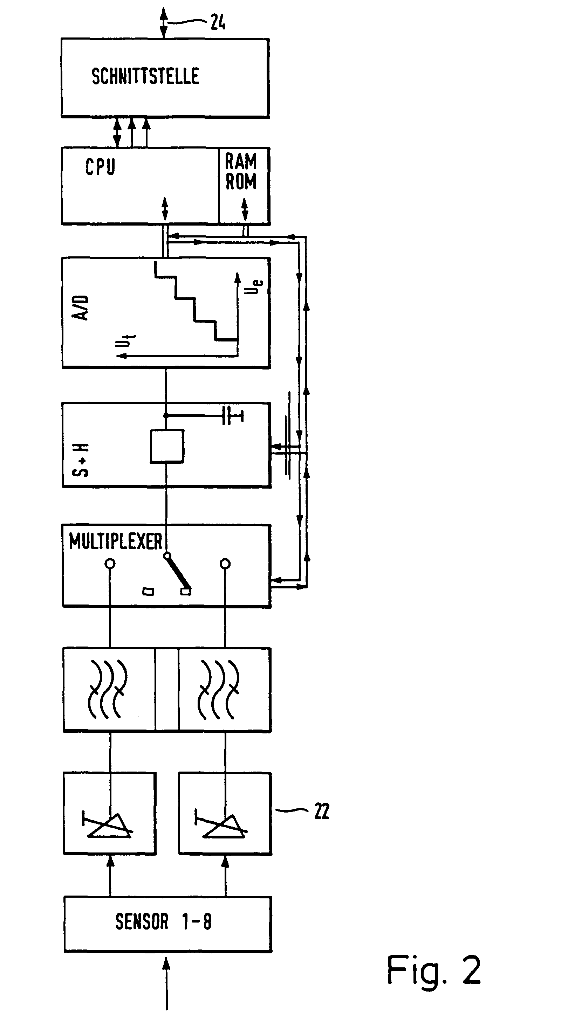

Gemäß dem Schaltbild in Fig. 2 wird der Ausgang aus den einzelnen Sensoren über eine Signalanpassung 22 und Kompensation an eine weitere Einheit mit Multiplexer-Taktgenerator, Meßstellenumschalter, Sample und Hold und A/D-Wandler gegeben. Hieran schließt sich eine Einheit bestehend aus einer Zentraleinheit (Kommunikationsprozessor) und der Schnittstelle mit Optokoppler an. Hieran schließt sich der Bus 24 an.

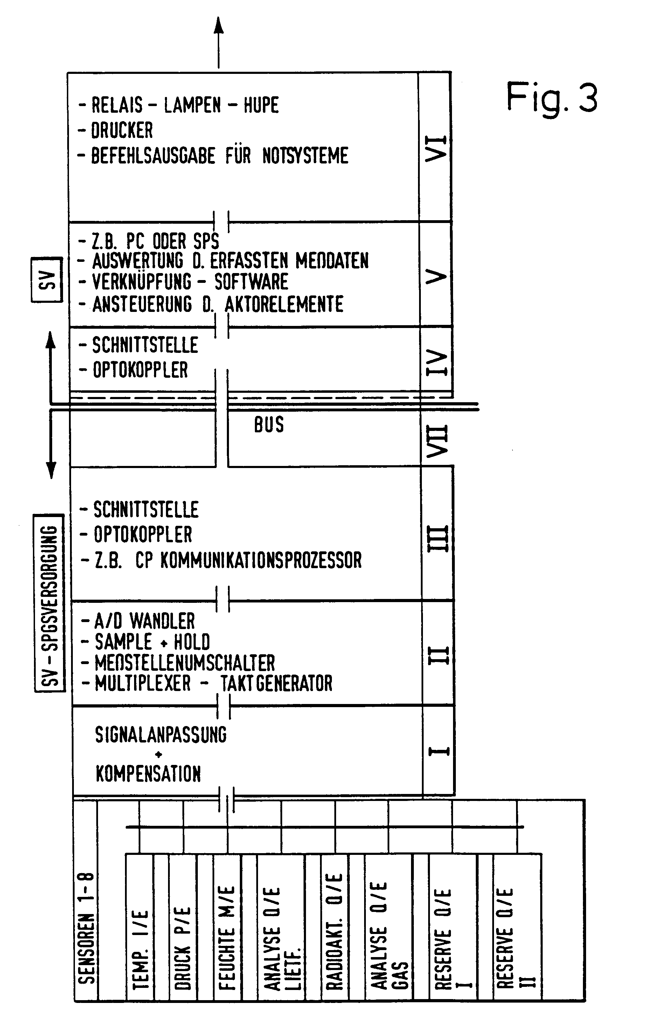

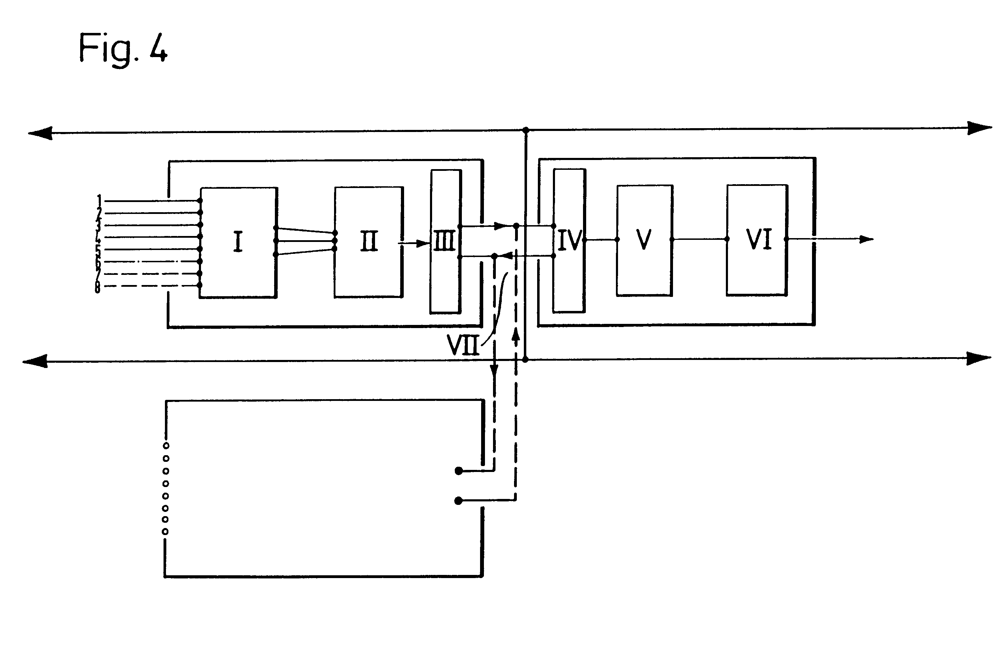

Die Daten werden an eine Einheit weitergeleitet, die über eine Schnittstelle mit Optokoppler ebenfalls an dem Bus angeschlossen ist und eine Datenverarbeitungsanlage enthält, die zur Auswertung der erfaßten Meßdaten dient. Diese Datenverarbeitungsanlage enthält weiterhin die Verknüpfungssoftware und dient auch zur Ansteuerung der Aktorelemente, die beispielsweise aus einem Relais, Lampen oder einer Hupe, einem Drucker oder einer Befehlsausgabe für Notsysteme bestehen (vgl. Fig. 3). Ein Gesamtüberblick des schematischen Aufbaus ist auch aus Fig. 4 zu ersehen. Dort sind die 8 Datenleitungen der entsprechenden Meßköpfe mit 1-8 bezeichnet. Diese Meßköpfe geben ihre Daten an die erste Baueinheit I der mikroelektronischen Schaltung weiter, die aus der Signalanpassung und Kompensation besteht. Diese Daten werden dann in die zweite Baueinheit der mikroelektronischen Schaltung 11 weitergegeben, die einen Multiplex-Taktgenerator, Meß-Stellenumschalter, Sample und Hold und A/B-Wandler enthält. Diese Daten werden dann an die erste Schnittstelle 111 weitergegeben.

Auf der anderen Seite des Systems befindet sich die zweite Schnittstelle IV, daran anschließend die Datenverarbeitungsanlage V und die Aktoren Vl. Zwischen der Schnittstelle 111 und der Schnittstelle IV ist das Bussystem VII (Bezugszeichen 24) angeordnet.

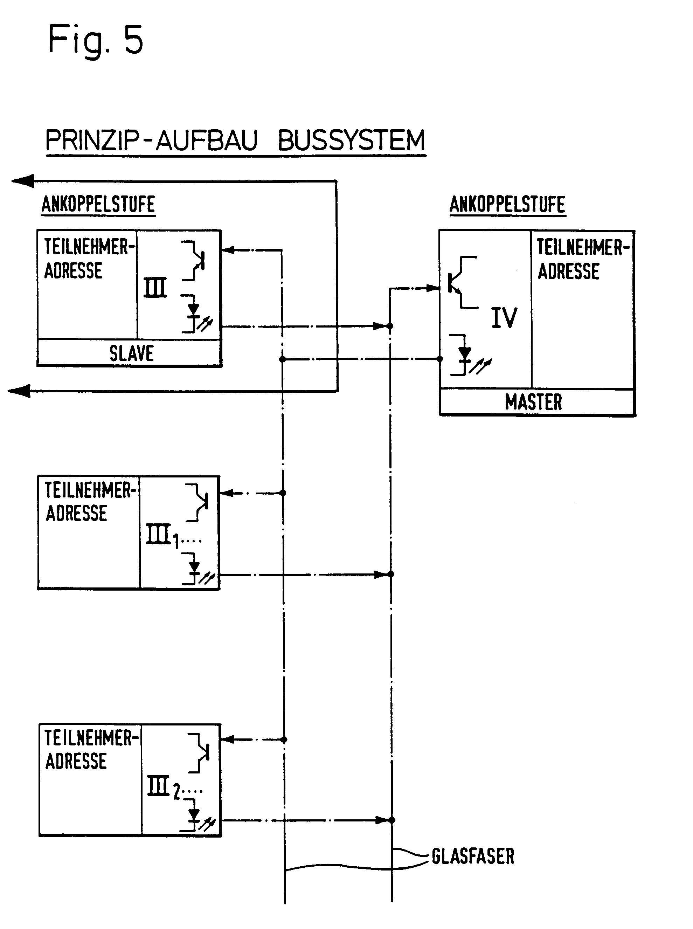

Der Prinzipaufbau des Bussystems ist in Fig. 5 dargestellt. Zwischen der ersten und zweiten Schnittstelle (Ankoppelstufen) arbeitet das Bussystem wie folgt: Die Teilnehmeradresse wird bei Master und Slave hardwaremäßig (Brücke) eingestellt. Der Master schickt ein Telegramm auf den Bus mit Teilnehmeradresse des anzusprechenden Slave. Wird die Adresse eines Slaves nicht quittiert (dreimal Anforderung durch den Master) wird durch den Master eine Störmeldung ausgelöst. Bei Negativfunktion der überwachten Quittung des Slaves schickt dieser ein Datentelegramm auf den Bus mit Adressenaufgabe des zu empfangenden Masters und den Nutzdaten (Sensorwerte).

Mit dem hier realisierten Bussystem ist eine ununterbrochene Ferndiagnose auf Anforderung im Taktzeitintervall über den jeweiligen Zustand der Altlast/Deponieabschottungssystematik in der physikalisch/chemischen Veränderungen bzw. des Verhaltens des Deponiegutes möglich. Die hohe Verfügbarkeit wird in bezug auf die Gesamtfläche durch das zuvor beschriebene Bussystem, welches anschlußintegriert angeordnet ist, hergestellt.

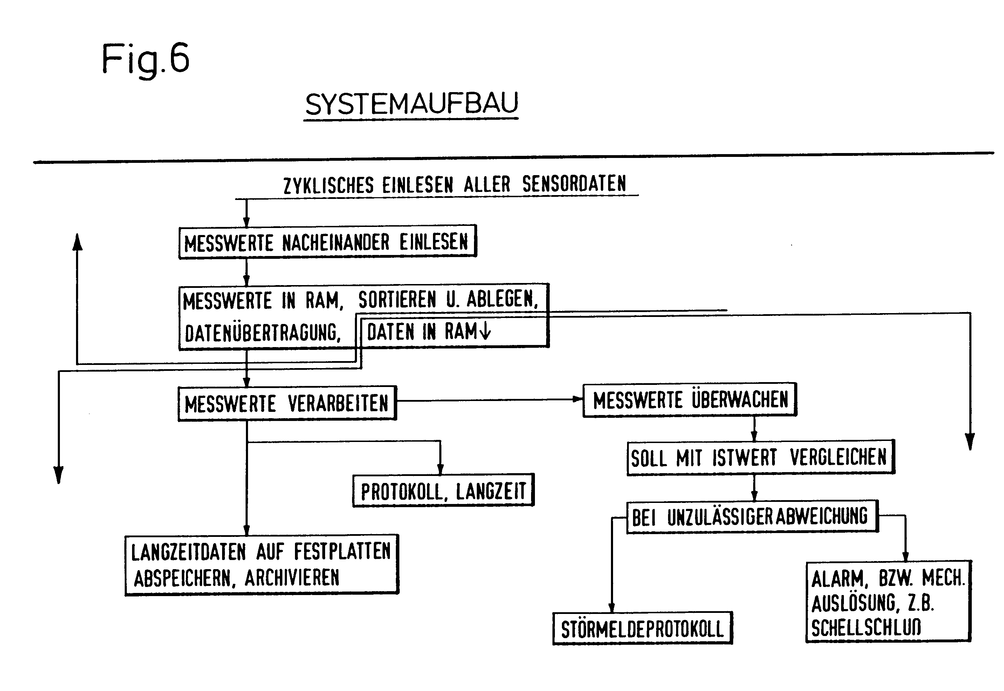

Anhand der Fig. 6 kann der Überwachungsvorgang mit dem erfindungsgemäßen System näher erläutert werden. Zunächst werden alle Sensordaten zyklisch eingelesen. Dabei werden die Meßwerte nacheinander eingelesen. Die Meßwerte werden sortiert und im RAM abgelegt. Dann werden die Meßwerte verarbeitet. Dabei wird einerseits ein Langzeitprotokoll angefertigt und es werden Langzeitdaten auf einer Festplatte abgespeichert und diese Festplatten können archiviert werden. Andererseits werden die Meßwerte überwacht und es wird der aktuelle Ist-Wert mit einem vorgegebenen Soll-Wert verglichen. Bei unzulässigen Abweichungen wird einerseits Alarm ausgegeben und andererseits wird ein Störmeldeprotokoll angefertigt.

Wie zuvor beschrieben wurde, ist anhand des erfindungsgemäßen Systems eine mikroelektronische Sensorüberwachung an die Hand gegeben, die das Geschehen in Altlasten-Deponien kontinuierlich überwacht. Die Sensoren erfassen Druck, Gas, Strahlung oder ähnliches, die in den nachgeordneten mikorelektronischen Bauteilen, die jedoch kompakt in einer Einheit zusammengefaßt sind, bestehend aus Sensorelementköpfen, Signalvorverarbeitung, Signalweiterverarbeitung, Schnittstelle und Bussystem, sowie weitere funktionsmikroelektronische Baugruppen, wie Schnittstelle, Datenauswertung/Aktoraussteuerung und Aktorelement, weiterverarbeitet werden.

Das anhand der Altlast-Deponien im Einzelnen erläuterte System der mikroelektronischen Sensorüberwachung läßt sich erfindungsgemäß auch in anderen Bereichen einsetzen. So kann es nicht nur in der Basis-Abdichtung von Deponien, sondern auch beispielsweise in der eventuell vorhandenen Oberflächenabdeckung einer Deponie integriert sein.

Es kann in anderer Ausführungsform auch in einem Müllgroßcontainer zur Identifikation der Zusammensetzung des Müllgutes installiert sein. Das System kann als integriertes System auch in einem zylindrischen Sensorgehäuse gekappselt sein. Es kann weiterhin in einer Spund- oder Schlitzwand eines Tunnelkörpers integriert sein, um Druckwasseransammlungen und Veränderungen des Gesteins- und Verfüllmaterials zu überwachen. Das System läßt sich auch in einem Kanalsanierungsrobotter integrieren, um bei den Sanierungsarbeiten von Kanälen Schadstoffbelastungen aufzuspüren bzw. zu überwachen. Schließlich läßt sich das System auch unterhalb von wasserführenden Kanälen und Rohren anordnen, um austretendes Wasser frühzeitig zu erkennen. Auch ähnliche Erkennungs-und Überwachungsaufgaben lassen sich vorteilhaft mit dem hier beschriebenen System der mikroelektronischen Sensorüberwachung lösen.

## Patentansprüche

1. System zur Erkennung und Überwachung von Schadstoffen,
dadurch gekennzeichnet,
daß in und/oder an mindestens einer Aufnahmekammer jeweils mindestens ein Sensorelement angeordnet ist,
daß innerhalb der mindestens einen Aufnahmekammer dem mindestens einen Sensorelement nachgeschaltet eine kompakte Mikroelektronikbaueinheit mit Signalanpassung, Signalverarbeitung und erster Schnittstelle angeordnet ist, daß über eine zweite Schnittstelle eine Datenverarbeitungsanlage zur Auswertung, Aktoraussteuerung und entsprechende Aktorelemente anschließbar sind und
daß ein Bussystem zwischen der ersten und zweiten Schnittstelle angeordnet ist.

2. System nach Anspruch 1, dadurch gekennzeichnet, daß die Sensoren mit diesen abdekkenden Sensorschutzhauben versehen sind.

3. System nach Anspruch 1 oder 2, dadurch gekennzeichnet, daß Sensoren zur Erfassung der Temperatur, Druck, Feuchte, Leitfähigkeit, Radioakitvität und/oder zur Analyse von Gas vorhanden sind, die taktweise nacheinander abfragbar sind.

4. System nach einem der Ansprüche 1-3, dadurch gekennzeichnet, daß die erste und zweite Schnittstelle ein Glasfaser-Optokopplersystem umfassen.

5. System nach einem der Ansprüche 1-4, dadurch gekennzeichnet, daß es zur Überwachung von Deponien mit einer Basis-Abdichtung, vorzugsweise einer Mehrkammerabschottungssperrfolie eingesetzt wird und daß die Aufnahmekammern verteilt über die Basis-Abdichtung angeordnet und in diese integriert sind.

6. System nach einem der Ansprüche 1-4, dadurch gekennzeichnet, daß es zur Überwachung von Deponien mit einer Oberflächenabdeckung verwendet wird, wobei die Aufnahmekammern verteilt über die Oberflächenabdekkung angeordnet und in diesen integriert sind.

7. System nach einem der Ansprüche 1-4, dadurch gekennzeichnet, daß es in einem Müllcontainer bzw. Müllbehältnis zur Identifikation der Zusammensetzung des Abfalls angeordnet ist.

8. System nach einem der Ansprüche 1-4, dadurch gekennzeichnet, daß mehrere Sensorelemente in einem vorzugsweise zylindrischen Sensorgehäuse zusammen mit der entsprechenden Elektronik angeordnet sind, wobei dieses gekappselte Sensorgehäuse in ein entsprechend vorgetriebenes Bohrloch absenkbar ist, um mit dem System in dem entsprechenden Bohrloch Daten über u. a. Feuchte, lonenkonzentration, Druck, Strahlung und Temperatur zu erfassen.

9. System nach Anspruch 8, dadurch gekennzeichnet, daß es mit einer tragbaren elektronischen Auswerteeinheit verbindbar ist.

10. System nach einem der Ansprüche 1 bis 4, dadurch gekennzeichnet, daß es in einer Spund- oder Schlitzwand innerhalb eines Tunnelkörpers angeordnet ist, um Druckwasseransammlungen und Veränderungen des Gesteins- und Verfüllmaterials zu erkennen.

11. System nach einem der Ansprüche 1 bis 4, dadurch gekennzeichnet, daß es in einen Kanalsanierungsrobotter integriert ist.

12. System nach einem der Ansprüche 1 bis 4, dadurch gekennzeichnet, daß es unterhalb von wasserführenden Kanälen und Rohren angeordnet ist, um austretendes Abwasser frühzeitig zu erkennen.
